# EUROPÄISCHE PATENTSCHRIFT

(11) **EP 1 549 155 B1**
(45) Veröffentlichungstag und Bekanntmachung des Hinweises auf die Patenterteilung: **02.06.2010**
(21) Anmeldenummer: 03769018.7
(22) Anmeldetag: 10.10.2003
(51) Int. Cl.: A23K 1/16, A23K 1/18, A61K 35/74

(54) **FUTTERMITTEL- UND/ODER TRINKWASSERZUSATZ FÜR NUTZTIERE**
FOOD ADDITIVE AND/OR DRINKING WATER ADDITIVE FOR DOMESTIC ANIMALS
ADDITIF ALIMENTAIRE ET/OU D'EAU POTABLE DESTINE A DES ANIMAUX UTILES

(30) Priorität: 11.10.2002 AT 15432002
(43) Veröffentlichungstag der Anmeldung: 06.07.2005
(73) Patentinhaber: Erber Aktiengesellschaft, 3130 Herzogenburg (AT)
(72) Erfinder: BINDER, Johann, A-7501 Unterwart (AT); BINDER, Eva-Maria, A-7501 Unterwart (AT); NITSCH, Sabine, A-3100 St. Pölten (AT); KLIMITSCH, Alfred, A-3454 Reidling (AT)
(74) Vertreter: Cunow, Gerda
(86) Internationale Anmeldenummer: PCT/AT2003/000308
(87) Internationale Veröffentlichungsnummer: WO 2004/032645

(56) Entgegenhaltungen:
- EP-A- 0 219 488
- WO-A-01/68085
- WO-A-02/39827
- WO-A-97/02757
- DE-U- 20 202 562
- P.D. MEYER ET AL.: "The immune effects of inulin in vitro and in vivo" AGRO-INDUSTRI HI-TECH., Bd. 11, Nr. 6, 2000, Seiten 18-20, XP008026714 TEKNOSCIENCE, MILAN., IT ISSN: 1120-6012
- DATABASE WPI Section Ch, Week 199232 Derwent Publications Ltd., London, GB; Class B04, AN 1992-265117 XP002267731 -& JP 04 182433 A (AJINOMOTO KK), 30. Juni 1992 (1992-06-30)
- F. LETTNER ET AL.: "Mikrobielle Leistungsförderer in der Schweinemast" FORDERUNGSDIENST, Bd. 40, Nr. 2, 1992, Seiten 41-45, XP008026716
- P.D. SCHLEY ET AL.: "The immune-enhancing effects of dietary fibres and prebiotics" BRITISH JOURNAL OF NUTRITION, Bd. 87, Nr. Suppl. 2, 2002, Seiten S221-S230, XP008026712
- DATABASE WPI Section Ch, Week 200280 Derwent Publications Ltd., London, GB; Class B04, AN 2002-737906 XP002267732 & KR 2002 043 078 A (HAN I K), 8. Juni 2002 (2002-06-08)

## Beschreibung

Die vorliegende Erfindung bezieht sich auf einen Futtermittel- und/oder Trinkwasserzusatz für Nutztiere, enthaltend wenigstens Bakterien des Stammes Streptococcus faecium IMB 52 (DSM 3530).

Die Verwendung von Bakterien als probiotische Komponenten in Futtermittel- und/oder Trinkwasserzusätzen für Nutztiere ist seit langem bekannt, beispielsweise aus der AT-B 383 856. In dieser AT-B 383 856 werden Stämme aus der Gruppe der Enterococcen, insbesondere der Stamm Streptococcus faecium IMB 52 (DSM 3530), dem Futtermittel beigemischt, um als mikrobielle Leistungsförderer zu wirken. Leistungsförderer sind hiebei Futtermittelzusatzstoffe, welche eine höhere Leistung, insbesondere eine höhere Fleischleistung der Schlachttiere in bezug auf die eingesetzte Futtermenge und Fütterungszeit bewirken. Darüber hinaus konnte gezeigt werden, daß durch Einsatz des Stammes Streptococcus faecium IMB 52 (DSM 3530) die Ansiedelung von pathogenen Bakterien und/oder Viren im Darm deutlich verringert werden kann und auch die Toxinbildung durch Pathogene.

Futtermittel- bzw. Trinkwasserzusätze zur Verbesserung der Immunität von Nutztieren werden seit längerer Zeit untersucht und getestet, um insbesondere während der Streßphasen von Nutztieren auftretende, besondere Belastungen für das Abwehrsystem des Organismus zu minimieren bzw. zu mildern. So sind besondere Streßbelastungen bei Jungtieren die Zeit des Absetzens bei Ferkeln und Umgruppierungen bzw. Transportüberbelastungen oder Futterumstellungen in der Kälbermast und Geflügelaufzucht. Auch jede chronische Erkrankung oder jede anhaltende Traumatisierung belastet als Streßfaktor den Organismus der Nutztiere und mindert die Leistung derselben. Während derartiger Streßphasen wird der Organismus der Tiere deutlich belastet, wobei sich jedoch auch zu jedem anderen Zeitpunkt das Tier in einer verdeckten Abwehrschwäche befinden kann und somit gegen die Einwirkung von sowohl potentiell pathogenen, wie pathogenen Keimen, und anderen Noxen besonders empfindlich ist. Vor allem können zu diesem Zeitpunkt Faktorenkrankheiten leichter Fuß fassen und es kann eine direkte Leistungsdepression bei Nutztieren eintreten, was eine nicht unbedeutende, wirtschaftliche Einbuße für den Tierhalter bedeutet.

Gegenwärtig wird hauptsächlich versucht, in den kritischen Phasen der Nutztierhaltung, wie beispielsweise dem Absetzen von Ferkeln oder den ersten Lebenstagen von Junghühnern, den Streß- bzw. Erkrankungsfaktoren durch die prophylaktische bzw. methaphylaktische Verabreichung von bakterioziden Antibiotika zu begegnen. Allerdings werden insbesondere für die Entfernung der wachstumsgehemmten Bakterien für Bakteriostatika und Chemotherapeutika a priori ein funktionierendes Abwehrsystem vorausgesetzt, welches naturgemäß während erhöhten Belastungsphasen von Nutztieren nicht immer vorhanden ist. Der Einsatz von Antibiotika hat neben der Unsicherheit des Gesundheitszustandes des behandelten Tieres auch den Nachteil, daß nach der Verabreichung von Antibiotika Wartezeiten existieren, welche eingehalten werden müssen, bevor ein Tier der Lebensmittelproduktion zugeführt werden kann. Die gesetzlich festgelegten Wartezeiten beziehen sich hiebei auf den Stoffwechsel von gesunden Tieren und es ist selbstverständlich, daß der Stoffwechsel eines kranken Tieres, welcher gegenüber demjenigen eines gesunden Tieres stark abweicht bzw. schwankt, oftmals nicht in der Lage ist, auch nach den angegebenen Wartezeiten die Antibiotika vollständig abzubauen. Es müssen daher vermehrte, genaue Rückstandskontrollen durchgeführt werden, um sicherzustellen, daß vom Tier stammende Lebensmittel keine Gefährdung des Konsumenten darstellen können.

Darüber hinaus muß damit gerechnet werden, daß ein großer Teil von Bakterienstämmen im Laufe der Zeit gegen die verabreichten Antibiotika resistent wird und daher die Wirkung dieser Substanzen nachläßt. In der Folge müßte die Dosierung der Antibiotika erhöht werden, was wiederum zu verlängerten Wartezeiten führt. Insgesamt ist festzuhalten, daß die Nachteile der Antibiotikagabe nicht nur in der Rückstandsproblematik und der Toxizität bzw. der Resistenz der Tiere liegen, sondern daß langfristig auch im Hinblick auf die Volksgesundheit von der Antibiotikagabe abgerückt werden sollte.

Des weiteren ist es aus der Literatur bekannt, daß diverse Kohlenhydrate bzw. Nicht-Stärke-Poly- oder -Oligosaccharide in der Diätetik eine bedeutende Rolle spielen. Es wird in diesem Zusammenhang beispielsweise auf die Literaturstelle mit dem Titel "Dietary Modulation of the Human Gut Microflora Using the Prebiotics Oligofructose and Inulin", Glenn R. Gibson, American Society for Nutritional Sciences, 1999, Seiten 1438S ff, verwiesen, in welcher unter anderem die Verwendung von Präbiotika in der Nahrung diskutiert und festgehalten wird, daß derartige Substanzen eine bessere Widerstandsfähigkeit gegen pathogene Keime und dgl. zur Verfügung stellen können.

Die vorliegende Erfindung zielt nun darauf ab, einen Futtermittel- und/oder Trinkwasserzusatz zur Verfügung zu stellen, der das angeborene Immunsystem von Nutz- bzw. Haustieren stärkt und gleichzeitig die Toxinbildung durch Pathogene im Darm verhindert bzw. bereits die Ansiedlung von Pathogenen hintanhält, so daß nicht nur eine erhöhte Leistung der Nutztiere erzielt wird, sondern auch die Ausfallsquote deutlich herabgesetzt wird.

Zur Lösung dieser Aufgabe ist der erfindungsgemäße Futtermittel- und/oder Trinkwasserzusatz im wesentlichen dadurch gekennzeichnet, daß zusätzlich standardisierte Zellwandbestandteile aus Bacillus sp. und/oder Streptoccocus sp. und/oder Bifidobacterium sp. sowie Inulin enthalten sind. Dadurch, daß in dem Futtermittel- und/oder Trinkwasserzusatz neben Streptococcus faecium IMB 52 (DSM 3530) als probiotische Komponente zusätzlich standardisierte Zellwandbestandteile, nämlich Bacillus sp. und/oder Streptoccocus sp. und/oder Bifidobacterium sp., enthalten sind, welche insbesondere eine immunstimulierende Kapazität durch Interaktion mit Oberflächenrezeptoren von Makrophagen aufweisen, wird eine verbesserte Makrophagenaktivität erzielt, wodurch insgesamt das angeborene Immunsystem gestärkt wird. Durch die verbesserte Makrophagenaktivität, insbesondere durch die gesteigerte Phagozytoseaktivität der Makrophagen, zeigen Tiere, welchen standardisierte Zellwandbestandteile verabreicht wurden, eine gesteigerte Resistenz gegenüber Infektionen und somit ein rascheres Wachstum und verringerte Ausfälle. Die gleichzeitige Verabreichung von einer probiotischen Komponente, nämlich Streptococcus faecium IMB 52 (DSM 3530), führt zu einer raschen Vermehrung im Darm und somit zu einer Besiedelung des Darms, wodurch vice versa die Ansiedlung von Pathogenen im Darm verhindert wird. Gleichzeitig wirkt Streptococcus faecium IMB 52 (DSM 3530) antagonistisch gegen Pathogene, so daß durch die Kombination der Verabreichung von Zellwandbestandteilen und der probiotischen Komponente einerseits die Resistenz gegenüber Infektionen deutlich gesteigert wird und andererseits gleichzeitig die Besiedelung des Darms mit Pathogenen verhindert bzw. behindert wird, wodurch insgesamt die Ausfallsquote von Tieren im Zuge der Aufzucht deutlich verringert werden konnte. Dadurch, daß darüber hinaus neben der probiotischen Komponente und den standardisierten Zellwandbestandteilen ein prebiotisches Fructooligosaccharid in dem Futtermittel- und/oder Trinkwasserzusatz enthalten ist, wird das Wachstum von Bifidobakterien, welche prebiotische Frauctooligosaccharide spezifisch als Nahrungsquelle nutzen können, stimuliert und somit die Besiedelung des Darms von Tieren mit natürlichen Darmbewohnern angeregt, wodurch wiederum die Ansiedelung von Pathogenen hintangehalten bzw. verhindert werden kann. Darüber hinaus kann durch die Verabreichung von prebiotischen Fructooligosacchariden das Darmfloragleichgewicht in Richtung positive Keime verschoben werden und insbesondere aufgrund der Wirkung der prebiotischen Fructooligosaccharide über den gesamten Magen-Darm-Bereich des Tieres ein Hochwandern von pathogenen Keimen in den Dünndarm hintangehalten werden. Auf diese Weise gelingt es ohne Verabreichung von Antibiotika die Besiedelung des Darms mit Pathogenen nahezu vollständig zu vermeiden.

Gemäß einer Weiterbildung der Erfindung sind die standardisierten Zellwandbestandteile aus dem Stamm Bacillus subtilis gewählt. Zellwandbestandteile aus dem genannten Stamm sind relativ einfach mit gängigen Herstellungsverfahren herstellbar bzw. inaktivierbar und können somit ökonomisch in größeren Mengen hergestellt werden. Darüber hinaus zeigen insbesondere Zellwandbestandteile des genannten Stamms besonders gute, immunstimulierende Kapazität durch Wechselwirkung mit Oberflächenrezeptoren von Makrophagen, so daß sie bevorzugt zum Einsatz gelangen.

Gemäß einer Weiterbildung sind die standardisierten Zellwandbestandteile aus Bacillus subtilis in einer Menge von 0,2 - 40 g/kg Futtermittel- und/oder Trinkwasserzusatz, insbesondere 5 - 15 g/kg, enthalten. Im Zuge von Reihenuntersuchungen konnte nachgewiesen werden, daß bei Einsatz der Bacillus subtilis als standardisierte Zellwandbestandteile in einer Menge von 0,2 - 40 g/kg Futtermittel- und/oder Trinkwasserzusatz gemeinsam mit dem Einsatz des Stammes Streptococcus faecium IMB 52 (DSM 3530) in Mengen von 1 x 107 bis 1 x 1017 cfu/kg ein synergistischer Effekt zwischen der probiotischen Komponente und den Zellwandbestandteilen erzielt werden konnte, welcher über die Sammelwirkung der Einzelbestandteile weit hinausging und somit die aktivierende Wirkung auf die Zellen deutlich gesteigert ist, was nur durch einen Synergismus der probiotischen Komponente mit Bacillus subtilis erklärbar ist.

Gemäß einer Weiterbildung ist das Inulin aus Lauch, Zwiebel, Knoblauch, Artischocke, Weizen, Zichorie, Topinambur, Tomate, Banane und/oder Gerste gewonnen, und ist in einer Menge von 100 - 950 g/kg des Futtermittel- und/oder Trinkwasserzusatzes enthalten. Gemäß einer Weiterbildung ist das Inulin in dem Futtermittelzusatz in einer Menge von 100 - 700 g/kg, insbesondere 320 - 540 g/kg, und in dem Trinkwasserzusatz in einer Menge von 530 - 950 g/kg, insbesondere 820 - 920 g/kg, enthalten. Insbesondere bei der Verwendung in Trinkwasserzusätzen ist Inulin als präbiotisch wirkende Fructooligosaccharide von besonderer Bedeutung, da es aufgrund seiner leichten Wasserlöslichkeit in großen Mengen zum Einsatz gelangen kann und somit eine extrem gute Stimulierung des Wachstums von Bifidobakterien im Magen-DarmTrakt von Tieren bewirken kann. Bei Einsatz von Inulin in Futtermittelzusätzen können geringere Mengen zum Einsatz gebracht werden, da, wie dies einer Weiterbildung der vorliegenden Erfindung entspricht, zusätzlich Meeres-Braunalgen als eine phycophytische Komponente, insbesondere in dem Futtermittel- und/oder Trinkwasserzusatz enthalten sind.

Gemäß einer Weiterbildung sind die Meeres-Braunalgen aus Ascophyllum nodosum gewählt. Meeresbraunalgen als phycophytische Komponenten verhalten sich in einem Futtermittel- und/oder Trinkwasserzusatz analog wie Zellwandbestandteile, wobei sie im Gegensatz zu Zellwandbestandteilen aufgrund der einfacheren Zugänglichkeit und Gewinnung in bedeutend höheren Mengen eingesetzt werden können. Erfindungsgemäß sind gemäß einer Weiterbildung die Meeresbraunalgen in einer Menge von 300 - 800 g/kg, insbesondere 450 - 550 g/kg, Futtermittelzusatz enthalten. Die Meeresbraunalgen weisen eine relativ geringe Wasserlöslichkeit auf, so daß ihr Einsatz in Trinkwasserzusätzen nur begrenzt möglich ist, und deshalb die erfindungsgemäß vorgeschlagenen, höheren Mengen lediglich in Futtermittelzusätzen zum Einsatz gelangen kann. Aufgrund der höheren, eingesetzten Mengen an phycophytischer Komponente kann daher in Futtermittelzusätzen die Menge an verwendeten Inulin herabgesetzt werden, da aufgrund immunstimulierenden Kapazität der phycophytischen Komponenten eine Ansiedelung von Pathogenen mit Sicherheit hintangehalten wird. Aus diesem Grund muß in diesem Fall keine erhöhte Aufmerksamkeit auf die Stimulierung des Wachstums von Bifidobakterien gelegt werden, so daß im Futtermittelzusatz deutlich geringere Mengen an Inulin als präbiotischen Fructooligosaccharide zum Einsatz gelangen können als im Trinkwasserzusatz.

Darüber hinaus zeigen die Meeresbraunalgen, als phycophytischen Komponenten, ebenso wie die Zellwandbestandteile mit der probiotischen Komponente einen synergistischen Effekt, welcher über die Summenwirkung der Einzelbestandteile hinausgeht, so daß bei Einsatz der phycophytischen Komponente gemeinsam mit Zellwandbestandteilen, nämlich Bacillus subtilis, und der probiotischen Komponente, nämlich Inulin, sowohl die Ansiedelung von Pathogenen im Magen-Darm-Bereich von Tieren als auch die Makrophagenaktivität derart verbessert werden kann, daß sowohl die Ausfallsquote von Tieren als auch deren Futterumwandlung deutlich gegenüber der Summe der Wirkung der Einzelbestandteile gesteigert werden konnte. Diese Steigerung fällt besonders deutlich aus, wenn, wie dies gemäß einer Weiterbildung der Erfindung vorgesehen ist, 10 - 50 % der Bakterien des Stammes Streptococcus faecium IMB 52 (DSM 3530) durch wenigstens einen weiteren Bakterienstamm ersetzt sind.

Gemäß einer Weiterbildung der Erfindung ist die wenigstens ein weiterer Bakterienstamm aus den Stämmen Bifidobacterium thermophilum (I-01) (DSM 14411), Bifidobacterium thermophilum (I-07) (DSM 14412), Bifidobacterium boum (I-12) (DSM 14413), Bifidobacterium thermophilum (I-15) (DSM 14414), Bifidobacterium thermophilum (I-19) (DSM 14415) und Bifidobacterium thermophilum (I-20) (DSM 14416) gewählt. Die aus den obengenannten Stämmen gewählten Bakterien zeigen hiebei mit dem Stamm Streptococcus faecium IMB 52 (DSM 3530) eine synergistische Wirkung aufgrund der verstärkten Ausnutzung des bifidogenen Effekts. Darüber hinaus wird die Ammoniumausscheidung der Tiere durch Unterdrückung der Ammonium-bildenden Bakterien verringert und das Ammonium verstärkt durch die Bifidusbakterien genutzt, wodurch eine weitere Stärkung des Immunsystems erzielt werden kann. Schließlich sind die Bakterien vom Stamm Bifidobakterium in der Lage, über die Produktion kurzkettiger Fettsäuren eine leicht absorbierbare, zusätzliche Energieressource für das Tier zur Verfügung zu stellen und auch bei der Produktion von Vitaminen sowie bei der Mineralabsorption durch verringerten pH-Wert und erhöhte Löslichkeit der Minerale in dem Magen-Darm-Saft deutliche Vorteile insbesondere im Hinblick auf die Futterumwandlung bzw. Futterausnutzung der Tiere zu bieten.

Gemäß einer Weiterbildung der Erfindung haben sich für die Aufzucht und Haltung von Tieren Futtermittel- und/oder Trinkwasserzusätze als besonders geeignet erwiesen, in welchen pro kg Futtermittel- und/oder Trinkwasserzusatz 8 g Bacillus subtilis, 60,0 g Streptococcus faecium IMB 52 (DSM 3530), 432 g Bifidobacterium thermophilum und Rest Ascophyllum nodosum enthalten sind, insbesondere jene, die pro kg Futtermittel- und/oder Trinkwasserzusatz 10 g Bacillus subtilis, 24,75 Streptococcus faecium IMB 52 (DSM 3530), 10,5 g Bifidobacterium thermophilum (DSM 14414) und Rest Inulin aus Zichorie, enthalten.

Die Erfindung wird anhand von Ausführungsbeispielen bzw. den beigeschlossenen Zeichnungen näher erläutert. In den Zeichnungen zeigt
Fig. 1 die synergistische Wirkungsweise eines Trinkwasserzusatzes, bestehend aus standardisierten Zellwandbestandteilen und einer probiotischen Komponente, nämlich Streptococcus faecium IBM 52 (DSM 3530), im Vergleich zu der Wirkung der Einzelbestandteile;
Fig. 2 die synergistische Wirkungsweise eines Futtermittelzusatzes, bestehend aus Bacillus sp. und/oder Streptococcus sp. und/oder Bifidobacterium sp. standardisierten Zellwandbestandteilen, einer probiotischen Komponente und Meeres-Braunalgen, im Vergleich zu der Wirkung der Einzelbestandteile;
Fig. 3 ein Diagramm eines Trinkwasserzusatzes, das die Ergebnisse der Testsubstanzen in Prozent der Phagozytose/Kontrolle, im Vergleich zu der Wirkung der Einzelbestandteile, zeigt; und
Fig. 4 ein Diagramm, das die Ergebnisse der Testsubstanzen in einem Futtermittelzusatz in Prozent der Phagozytose/Kontrolle, im Vergleich zu der Wirkung der Einzelbestandteile, zeigt.

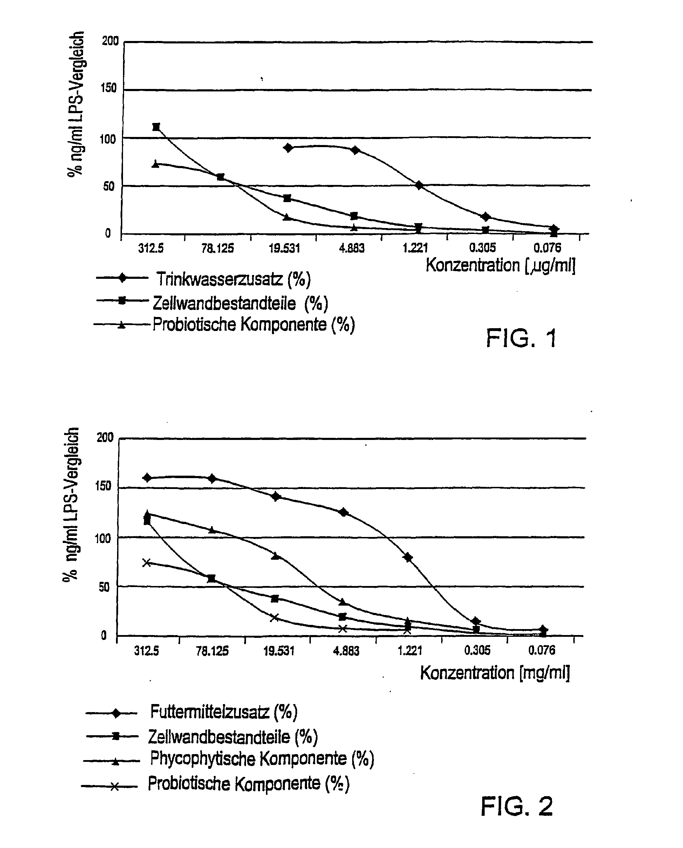

In dem Diagramm von Fig. 1 sind die Ergebnisse von Versuchen betreffend die Wirkungsweise von Produkten gemäß der vorliegenden Erfindung gegenüber der Wirkungsweise der Einzelsubstanzen als Konzentrationen gegen % Positivkontrolle aufgezeichnet, woraus sich ergibt, daß zwar die Einzelsubstanzen stimulierend auf Makrophagen wirken, jedoch die Kombination, insbesondere im Konzentrationsbereich von etwa 0,2 - 19,5 µg/ml, einen synergistischen Effekt zeigt, welcher über die Summenwirkung der Einzelkomponenten weit hinausgeht. Es ist somit mit einem Trinkwasserzusatz, der aus standardisierten Zellwandbestandteilen und einer probiotischen Komponente zusammengesetzt ist und welchem zusätzlich Inulin beigemischt ist, möglich, die Makrophagen im Magen-Darm-Trakt extrem zu stimulieren und die Ansiedelung von Pathogenen hinanzuhalten.

Das weiters in dem Trinkwasserzusatz enthaltene Inulin als präbiotisches Fructooligosaccharid trägt hier zu dem synergistischen Effekt nichts bei, da es lediglich das Wachstum der Bifidobakterien stimuliert und somit einen bifidogenen Effekt zeigt, welcher zwar ein erwünschter Zusatzeffekt ist, jedoch in bezug auf die Makrophagenaktivität bzw. das Verhindern der Ansiedelung von Pathogenen nicht relevant ist.

Fig. 2 zeigt in einem analogen Diagramm die synergistische Wirkungsweise eines Futtermittelzusatzes, welcher neben der probiotischen Komponente und den standardisierten Zellwandbestandteilen auch die phycophytische Komponente enthält. Aus Fig. 2 kann hiebei deutlich entnommen werden, daß hier der synergistische Effekt, insbesondere bei Konzentrationen von etwa 0,5 - 5 mg/ml, extrem hoch ist und somit der Einsatz von geringen Mengen des erfindungsgemäßen Futtermittelzusatzes extrem gute Ergebnisse bei der Vermeidung der Ansiedelung von Pathogenen bzw. eine gute Stimulierung der Makrophagen bewirkte.

Für die Beurteilung der Mischungen wurden die Einzelsubstanzen und die jeweiligen Substanzkombinationen gemeinsam getestet und anschließend die Ergebnisse miteinander verglichen. Bei diesen Untersuchungen konnte ebenfalls gezeigt werden, daß der Futtermittelzusatz und der Trinkwasserzusatz die Phagozytose-Aktivität der Zellen wirkungsvoller steigern als die summierten Effekte der Einzelsubstanzen. Somit konnte auch in diesem Testsystem eine synergistische Wirkung der Komponenten in dem Futtermittelzusatz und Trinkwasserzusatz nachgewiesen werden.

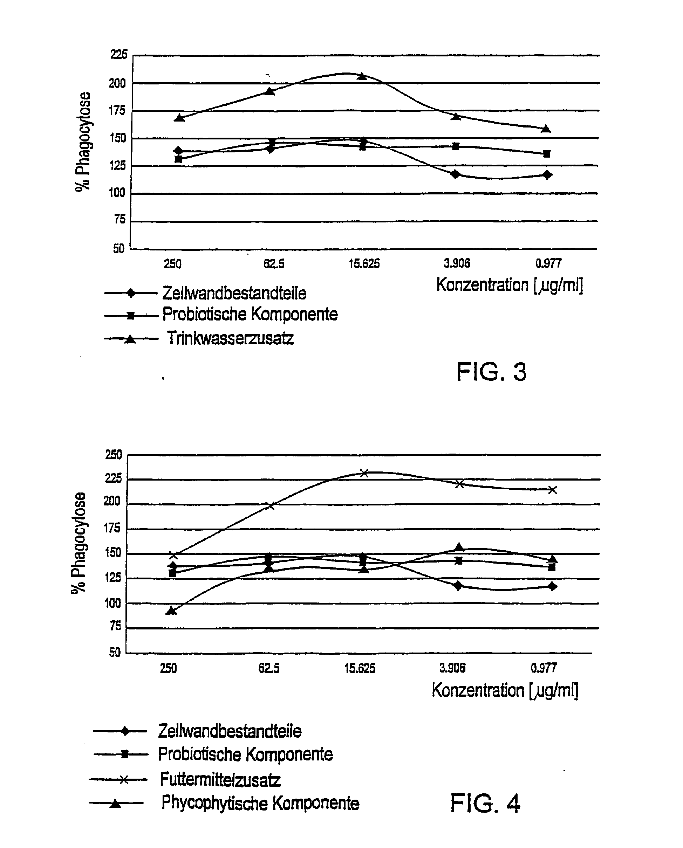

Die Konzentrationsbereiche für den Einsatz der einzelnen Substanzen, um die gewünschten, synergistischen Effekte gemäß der Erfindung zu erzielen, wurden in in vitro Versuchen mit einem Zellkultur-Testsystem ermittelt. Hiebei wurden unterschiedliche Substanzen, beispielsweise Streptococcus faecium IMB 52 (DSM 3530), Bacillus subtilis Zellwände, probiotische Komponenten und phycophytische Komponente, dem Testsystem in den in den Figuren 3 und 4 angegebenen Konzentrationen eingesetzt und die aufgezeigten Effekte erzielt. Nachfolgend wurden diese Erkenntnis auf in vivo Fütterungsversuche umgesetzt, wobei bei Beibehaltung der angegebenen Verhältnisse folgende Ergebnisse erzielt wurden: So ergibt sich aus einer optimalen Einsatzmenge für Zellwandbestandteile von 0,2 - 19,5 µg/ml in vitro im Fertigfutter eine Einsatzmenge von 0,2 - 20 g/t. Diese Menge an Substanz muß in einem kg des Futtermittelzusatzstoffes vorhanden sein, was heißt, daß der Zusatzstoff bei einer Dosierung von 1 kg/t Fertigfutter die Substanzen in 1000-facher Konzentration enthalten muß. Daraus ergibt sich eine Menge von 0,2 - 20 g Zellwandbestandteile pro kg Futtermittelzusatz. Analog dazu errechnen sich die Mengen für die prebiotische und die phycophytische Komponente, wobei sich aus weiteren Untersuchungen für den Einsatz in vivo eine höhere Einsatzmenge dieser beiden Komponenten um einen Faktor 25 bis 35 ergab. Dieser Faktor berücksichtigt die in vivo im Darm pro g oder ml vorhandene, größere Menge an Mikroorganismen im Vergleich zum in vitro Testsystem. Daraus ergeben sich die angegebenen Mengen für die probiotische und die phycophytische Komponente im Futtermittel- und/oder Trinkwasserzusatz. Im Gegensatz dazu wird die Menge an probiotischer Komponente über die Anzahl an koloniebildenden Einheiten bestimmt.

### Trinkwasserzusatz

### Werte in % (Differenz Ergebnisse der Testsubstanzen in % Phagozytose/Kontrolle)

| [µg/ml] | Zellwandteile Bacillus subtilis (%) | Probiot. Komponente (%) | ∑ Komponenten | Trinkwasserzusatz | Synergie-Effekt |
|---|---|---|---|---|---|
| 250 | 38,00 | 34,40 | 68,40 | 67,71 | nein |
| 62,5 | 41,02 | 46,39 | 87,41 | 90,51 | ja |
| 15,625 | 46,26 | 41,99 | 88,25 | 104,30 | ja |
| 3,906 | 16,60 | 40,60 | 57,20 | 70,40 | ja |
| 0,977 | 15,50 | 33,90 | 49,40 | 61,70 | ja |

### Futtermittelzusatz

### Werte in % (Differenz Ergebnisse der Testsubstanzen in % Phagozytose/Kontrolle)

| [µg/ml] | Ascophyllum nodosum feines Pulver (%) | Zellwandteile Bacillus subtilis (%) | Probiot. Komponente (%) | ∑ Komponenten | Futtermittelzusatz | Synergie-Effekt |
|---|---|---|---|---|---|---|
| 250 | -4,92 | 38,00 | 34,40 | 63,48 | 48,18 | nein |
| 62,5 | 35,61 | 41,02 | 46,39 | 123,02 | 98,40 | nein |
| 15,625 | 37,57 | 46,26 | 41,99 | 125,82 | 130,12 | ja |
| 3,906 | 52,55 | 16,60 | 40,60 | 10,9,75 | 117,61 | ja |
| 0,977 | 41,38 | 15,50 | 33,90 | 90,78 | 112,21 | ja |

Anhand von Versuchen wurden die erfindungsgemäßen Futtermittel- bzw. Trinkwasserzusätze an Küken getestet, wobei gemäß Versuch 1 ein Trinkwasserzusatz an Küken verabreicht wurde, gemäß Versuch 2 ein Futtermittelzusatz an Küken verabreicht wurde, gemäß Versuch 3 eine Kombination aus Trinkwasser- und Futtermittelzusatz an Küken verabreicht wurde und in Versuch 4 eine Kombination aus Futtermittel- und Trinkwasserzusatz bei einem Salmonellen-Challenge an Küken verabreicht wurde.

### Versuch 1

1.400 Broilerküken (50 % männlich, 50 % weiblich) wurden vom 1. bis 42. Lebenstag überwacht und in Gruppen von je 280 Tieren eingeteilt. Den Tieren wurde Trinkwasser und Futter ad libitum zur Verfügung gestellt. Jene Gruppen von Tieren, die einen Trinkwasserzusatz erhielten, erhielten diesen am 1. Tag durch Versprühen des Trinkwasserzusatzes, an den Tagen 2 und 3 durch Trinkwasserapplikation.

Die Gruppeneinteilung war wie folgt: eine Kontrollgruppe erhielt keinerlei Trinkwasserzusatz; Gruppe 1 erhielt einen Trinkwasserzusatz, bestehend aus Zellwandbestandteilen von Bacillus subtilis und prebiotischem Fructooligosaccharid; Gruppe 2 erhielt Streptococcus faecium IMB 52 (DSM 3530) und prebiotisches Fructooligosaccharid; Gruppe 3 erhielt Bacillus subtilis Zellwandbestandteile, Streptococcus faecium IMB 52 (DSM 3530) und prebiotisches Fructooligosaccharid; und Gruppe 4 erhielt Bacillus subtilis Zellwandbestandteile plus Streptococcus faecium IMB 52 (DSM 3530) plus prebiotisches Fructooligosaccharid plus Bifidobakterien. Die Lebendmasseentwicklung der Broiler ebenso wie ihre Sterblichkeit sind in den unten angeführten Tabellen gezeigt. Aus diesen Untersuchungen läßt sich eindeutig erkennen, daß jene Tiere, die sowohl Zellwandbestandteile von Bacillus subtilis als auch Streptococcus faecium IMB 52 (DSM 3530) erhielten, sowohl in der Lebendmasseentwicklung als auch in der Mortalität sämtlichen anderen Gruppen deutlich überlegen waren.

### Lebendmasseentwicklung (g)

| | Kontrolle | Gruppe 1 | Gruppe 2 | Gruppe 3 | Gruppe 4 |
|---|---|---|---|---|---|
| Tag 1 | 45,1 | 45,0 | 44,6 | 44,8 | 45,1 |
| Tag 4 | 65,3 | 696,0 | 66,7 | 67,9 | 69,3 |
| Tag 7 | 135,7 | 136,4 | 139,4 | 137,2 | 140,4 |
| Tag 14 | 306,8 | 310,7 | 318,3 | 320,6 | 328,0 |
| Tag 21 | 599,1 | 599,0 | 603,7 | 610,2 | 624,3 |
| Tag 35 | 1396,5 | 1406,3 | 1433,4 | 1420,5 | 1434,8 |
| Tag 42 | 1865,4 | 1873,6 | 1888,2 | 1892,7 | 1918,4 |

### Mortalität (%)

| | Kontrolle | Gruppe 1 | Gruppe 2 | Gruppe 3 | Gruppe 4 |
|---|---|---|---|---|---|
| Tag 4 | 0 | 0 | 0 | 0 | 0 |
| Tag 7 | 1,14 | 1,14 | 0,98 | 0,86 | 0,57 |
| Tag 14 | 2,20 | 2,00 | 2,00 | 2,00 | 1,71 |
| Tag 21 | 2,57 | 2,57 | 2,31 | 2,29 | 2,29 |
| Tag 35 | 5,14 | 5,18 | 4,57 | 4,00 | 4,00 |
| Tag 42 | 6,29 | 6,36 | 6,19 | 6,12 | 6,00 |

### Versuch 2

1.250 Broilerküken (50 % männlich, 50 % weiblich) wurden vom 1. bis 42. Lebenstag überwacht und in Gruppen von je 250 Tieren eingeteilt. Die Tiere erhielten ein Starterfutter vom 1. bis zum 21. Tag, weiters wurde ihnen Trinkwasser und Futter ad libitum zur Verfügung gestellt.

Die Gruppeneinteilung war wie folgt: eine Kontrollgruppe erhielt keinerlei Trinkwasserzusatz; Gruppe 1 erhielt einen Futtermittelzusatz, bestehend aus Zellwandbestandteilen von Bacillus subtilis, und prebiotischem Fructooligosaccharid; Gruppe 2 erhielt Streptococcus faecium IMB 52 (DSM 3530) und prebiotisches Fructooligosaccharid; Gruppe 3 erhielt Bacillus subtilis Zellwandbestandteile, Streptococcus faecium IMB 52 (DSM 3530) und prebiotisches Fructooligosaccharid; Gruppe 4 erhielt Bacillus subtilis Zellwandbestandteile plus Streptococcus faecium IMB 52 (DSM 3530) plus prebiotisches Fructooligosaccharid plus Ascophyllum nodosum; und Gruppe 5 erhielt Bacillus subtilis Zellwandbestandteile plus Streptococcus faecium IMB 52 (DSM 3530) plus prebiotisches Fructooligosaccharid plus Ascophyllum nodosum plus Bifidobakterien. In Versuch 2 wurde sowohl Lebendgewichtentwicklung der Broiler ebenso wie ihre Mortalität und ihre Futterverwertung untersucht. Aus diesem Versuch läßt sich deutlich ersehen, daß die Gruppen 3 bis 5, welche sowohl Bacillus subtilis Zellwandbestandteile als auch Streptococcus faecium IMB 52 (DSM 3530) und andere Komponenten erhielten, sowohl in der Lebendgewichtentwicklung als auch in der Mortalität und der Futterverwertung sämtlichen anderen Gruppen deutlich überlegen waren.

### Lebendgewichtentwicklung (g)

| | Kontrolle | Gruppe 1 | Gruppe 2 | Gruppe 3 | Gruppe 4 | Gruppe 5 |
|---|---|---|---|---|---|---|
| Tag 7 | 134 | 134 | 133 | 136 | 138 | 136 |
| Tag 14 | 280 | 278 | 278 | 284 | 285 | 288 |
| Tag 21 | 572 | 576 | 574 | 580 | 586 | 591 |
| Tag 35 | 1613 | 1630 | 1634 | 1660 | 1663 | 1674 |
| Tag 42 | 2121 | 2145 | 2156 | 2179 | 2174 | 2198 |

### Mortalität (%)

| | Kontrolle | Gruppe 1 | Gruppe 2 | Gruppe 3 | Gruppe 4 | Gruppe 5 |
|---|---|---|---|---|---|---|
| Tag 7 | 0,86 | 1,14 | 1,14 | 0,57 | 0,64 | 0,49 |
| Tag 14 | 2,00 | 2,00 | 2,00 | 1,71 | 1,65 | 1,53 |
| Tag 21 | 2,57 | 2,48 | 2,42 | 2,29 | 2,29 | 2,29 |
| Tag 35 | 5,14 | 4,57 | 4,63 | 4,00 | 4,14 | 3,52 |
| Tag 42 | 6,29 | 6,14 | 6,14 | 6,00 | 6,05 | 5,85 |

### Futterverwertung (FCR; g/g)

| | Kontrolle | Gruppe 1 | Gruppe 2 | Gruppe 3 | Gruppe 4 | Gruppe 5 |
|---|---|---|---|---|---|---|
| Tag 7 | 1,42 | 1,41 | 1,39 | 1,38 | 1,38 | 1,35 |
| Tag 14 | 1,60 | 1,58 | 1,59 | 1,57 | 1,58 | 1,56 |
| Tag 21 | 1,71 | 1,69 | 1,70 | 1,67 | 1,68 | 1,65 |
| Tag 35 | 1,78 | 1,76 | 1,73 | 1,72 | 1,70 | 1,71 |
| Tag 42 | 1,90 | 1,88 | 1,85 | 1,81 | 1,80 | 1,76 |

### Versuch 3

1.750 Broilerküken (50 % männlich, 50 % weiblich) wurden vom 1. bis 49. Lebenstag überwacht und in Gruppen von je 350 Tieren eingeteilt. Die Tiere der Gruppe 1 erhielten einen Trinkwasserzusatz, Gruppe 2 erhielt einen Futtermittelzusatz und Gruppe 3 erhielt eine Kombination aus Trinkwasser- und Futtermittelzusatz, weiters wurde ihnen Trinkwasser und Futter ad libitum zur Verfügung gestellt.

Die Gruppeneinteilung war wie folgt: eine Kontrollgruppe erhielt keinerlei Futtermittel- bzw. Trinkwasserzusatz; Gruppe 2 erhielt Streptococcus faecium IMB 52 (DSM 3530) und prebiotisches Fructooligosaccharid; und Gruppe 3 erhielt Bacillus subtilis Zellwandbestandteile, Streptococcus faecium IMB 52 (DSM 3530) und prebiotisches Fructooligosaccharid. Hieraus läßt sich deutlich ersehen, daß jene Gruppe von Tieren, die sowohl Futtermittel- als auch Trinkwasserzusatz erhielten, sowohl in der Lebendgewichtentwicklung als auch in der Mortalität sämtlichen anderen Gruppen deutlich überlegen waren, wobei diese Überlegenheit über die Summe der Wirkung der Einzelbestandteile hinausging.

### Lebendgewicht (g)

| | Kontrolle | Gruppe 1 | Gruppe 2 | Gruppe 3 |
|---|---|---|---|---|
| Tag 7 | 130,7 | 139,4 | 137,2 | 140,4 |
| Tag 14 | 407,4 | 414,2 | 417,4 | 422,8 |
| Tag 21 | 838,6 | 849,8 | 855,6 | 862,5 |
| Tag 35 | 1698,4 | 1719,2 | 1715,4 | 1711,8 |
| Tag 49 | 2190,6 | 2183,8 | 2203,6 | 2295,5 |

### Mortalität (%)

| | Kontrolle | Gruppe 1 | Gruppe 2 | Gruppe 3 |
|---|---|---|---|---|
| Tag 7 | 1,60 | 0,66 | 0,66 | 0,40 |
| Tag 14 | 2,00 | 1,66 | 1,66 | 1,20 |
| Tag 21 | 4,33 | 2,80 | 2,88 | 2,40 |
| Tag 35 | 5,60 | 4,90 | 3,77 | 30,3 |
| Tag 49 | 6,80 | 6,10 | 5,90 | 4,80 |

### Versuch 4

450 Broilerküken (50 % männlich, 50 % weiblich) wurden vom 1. bis 49. Lebenstag überwacht und in Gruppen von je 90 Tieren eingeteilt. Den Tieren wurde am Tag 1 ein Trinkwasserzusatz durch Sprühen, an den Tagen 2 und 3 ein Trinkwasserzusatz über das Trinkwasser und ein Futtermittelzusatz über das Starterfutter für die Dauer von 21 Tagen verabreicht. 24 h nach der Verabreichung des Trinkwasserzusatzes wurden die Tiere mit 0,25 ml Salmonella enteritidis (4 x 104 cfu/ml) inokuliert.

Die Gruppeneinteilung war wie folgt: eine Kontrollgruppe (+) erhielt keinerlei Futtermittel- bzw. Trinkwasserzusatz, wurde jedoch inokuliert; Gruppe 1 erhielt Bacillus subtilis Zellwandbestandteile plus Streptococcus faecium IMB 52 (DSM 3530) plus prebiotisches Fructooligosaccharid plus Bifidobakterien; Gruppe 2 erhielt Bacillus subtilis Zellwandbestandteile plus Streptococcus faecium IMB 52 (DSM 3530) plus prebiotisches Fructooligosaccharid plus Ascophyllum nodosum plus Bifidobakterien; Gruppe 3 erhielt eine Kombination aus Trinkwasser- und Futtermittelzusatz; und eine Kontrollgruppe (-) erhielt keinerlei Futtermittel- bzw. Trinkwasserzusatz und wurde auch nicht inokuliert. Aus diesem Versuch ist eindeutig zu erkennen, daß bei Tieren, die einen Futtermittel- und/oder Trinkwasserzusatz gemäß der Erfindung erhielten, der Organbefall mit Salmonella enteritidis deutlich gegenüber der Kontrollgruppe, die keinen Futtermittel- und/oder Trinkwasserzusatz erhielt, abgesenkt werden konnte, wobei jene Tiere, die sowohl Futtermittelals auch Trinkwasserzusatz erhielten, am Ende des Zusatzes keinerlei Organbefall mehr zeigten. Zusammenfassend ist festzuhalten, daß es mit dem erfindungsgemäßen Futtermittel- und/oder Trinkwasserzusatz gelingt, nicht nur das Lebendmassegewicht der Tiere deutlich zu steigern und ihre Mortalität abzusenken, sondern auch den Organbefall mit pathogenen Keimen nahezu vollständig zu unterdrücken.

| | Kontrolle (+) | Gruppe 1 | Gruppe 2 | Gruppe 3 | Kontrolle (-) |
|---|---|---|---|---|---|
| Tag 7 | 10/15 | 3/15* | 3/15* | 1/15* | 0/15 |
| Tag 14 | 9/15 | 2/15* | 2/15* | 1/15* | 0/15 |
| Tag 21 | 8/15 | 2/15* | 2/15* | 0/15* | 0/15 |
| Tag 35 | 7/15 | 0/15* | 1/15* | 0/15* | 0/15 |
| Tag 49 | 5/15 | 0/15* | 0/15* | 0/15* | 0/15 |

| | | | | | |
|---|---|---|---|---|---|
| * ... signifikanter Unterschied (P < 0,05) | | | | | |

## Patentansprüche

1. Futtermittel- und/oder Trinkwasserzusatz für Nutztiere, enthaltend wenigstens Bakterien des Stammes Streptococcus faecium IMB 52 (DSM 3530), **dadurch gekennzeichnet, daß** zusätzlich standardisierte Zellwandbestandteile aus Bacillus sp. und/oder Streptoccocus sp. und/oder Bifidobacterium sp. sowie Inulin enthalten sind.

2. Futtermittel- und/oder Trinkwasserzusatz nach Anspruch 1, **dadurch gekennzeichnet, daß** die standardisierten Zellwandbestandteile aus dem Stamm Bacillus subtilis gewählt sind.

3. Futtermittel- und/oder Trinkwasserzusatz nach Anspruch 1 oder 2, **dadurch gekennzeichnet, daß** die standardisierten Zellwandbestandteile aus Bacillus subtilis in einer Menge von 0,2 - 40 g/kg Futtermittel- und/oder Trinkwasserzusatz, insbesondere 5 - 15 g/kg, enthalten sind.

4. Futtermittel- und/oder Trinkwasserzusatz nach Anspruch 1, **dadurch gekennzeichnet, daß** Inulin aus Lauch, Zwiebel, Knoblauch, Artischocke, Weizen, Zichorie, Topinambur, Tomate, Banane und/oder Gerste gewonnen ist, und in einer Menge von 100 - 950 g/kg des Futtermittel- und/oder Trinkwasserzusatzes enthalten ist.

5. Futtermittel- und/oder Trinkwasserzusatz nach Anspruch 4, **dadurch gekennzeichnet, daß** Inulin im Futtermittelzusatz in einer Menge von 100 - 700 g/kg, insbesondere 320 - 540 g/kg, enthalten ist.

6. Futtermittel- und/oder Trinkwasserzusatz nach Anspruch 4, **dadurch gekennzeichnet, daß** Inulin im Trinkwasserzusatz in einer Menge von 530 - 950 g/kg, insbesondere 820 - 920 g/kg, enthalten ist.

7. Futtermittel- und/oder Trinkwasserzusatz nach einem der Ansprüche 1 - 6, **dadurch gekennzeichnet, daß** die Bakterien des Stammes Streptococcus faecium IMB 52 (DSM 3530) teilweise durch wenigstens einen weiteren Bakterienstamm ersetzt sind.

8. Futtermittel- und/oder Trinkwasserzusatz nach einem der Ansprüche 1 - 7, **dadurch gekennzeichnet, daß** 10 - 50 % der Bakterien des Stammes Streptococcus faecium IMB 52 (DSM 3530) durch wenigstens einen weiteren Bakterienstamm ersetzt sind.

9. Futtermittel- und/oder Trinkwasserzusatz nach einem der Ansprüche 1 - 8, **dadurch gekennzeichnet, daß** wenigstens ein weiterer Bakterienstamm aus den Stämmen Bifidobacterium thermophilum (I-01) (DSM 14411), Bifidobacterium thermophilum (I-07) (DSM 14412), Bifidobacterium boum (I-12) (DSM 14413), Bifidobacterium thermophilum (I-15) (DSM 14414), Bifidobacterium thermophilum (I-19) (DSM 14415) und Bifidobacterium thermophilum (I-20) (DSM 14416) gewählt ist.

10. Futtermittel- und/oder Trinkwasserzusatz nach einem der Ansprüche 1 - 9, **dadurch gekennzeichnet, daß** zusätzlich Meeres-Braunalgen enthalten ist.

11. Futtermittel- und/oder Trinkwasserzusatz nach Anspruch 9, **dadurch gekennzeichnet, daß** die Meeres-Braunalgen Ascophyllum nodosum sind.

12. Futtermittel- und/oder Trinkwasserzusatz nach Anspruch 10 oder 11, **dadurch gekennzeichnet, daß** Ascophyllum nodosum in einer Menge von 300 - 800 g/kg, insbesondere 450 - 550 g/kg, Futtermittelzusatz enthalten ist.

13. Futtermittel- und/oder Trinkwasserzusatz nach einem der Ansprüche 1 - 12, **dadurch gekennzeichnet, daß** pro kg Futtermittel- und/oder Trinkwasserzusatz 8 g Bacillus subtilis, 60,0 g Streptococcus faecium IMB 52 (DSM 3530), 432 g Bifidobacterium thermophilum und Rest Ascophyllum nodosum enthalten sind.

14. Futtermittel- und/oder Trinkwasserzusatz nach einem der Ansprüche 1 - 12, **dadurch gekennzeichnet, daß** pro kg Futtermittel- und/oder Trinkwasserzusatz 10 g Bacillus subtilis, 24,75 g Streptococcus faecium IMB 52 (DSM 3530), 10,5 Bifidobacterium thermophilum und Rest Inulin, insbesondere aus zichorie, enthalten ist.

## Claims

1. A feed additive and/or drinking water additive for domestic animals, which contains at least bacteria of the strain *Streptococcus faecium* IMB 52 (DSM 3530), **characterized in that** standardized cell wall components from Ba*cillus sp.* and/or *Streptoccocus sp.* and/or *Bifidobacterium sp*. as well as inulin are additionally contained.

2. A feed additive and/or drinking water additive according to claim 1, **characterized in that** the standardized cell wall components are selected from the strain *Bacillus subtilis*.

3. A feed additive and/or drinking water additive according to claim 1 or 2, **characterized in that** the standardized cell wall components from *Bacillus subtilis* are contained in an amount of from 0,2 to 40 g/kg feed additive and/or drinking water additive and, in particular, 5 to 15 g/kg.

4. A feed additive and/or drinking water additive according to claim 1, **characterized in that** inulin is isolated from leek, onion, garlic, artichoke, wheat, chicory, topinambour, tomato, banana and/or rye and is contained in an amount of from 100 to 950 g/kg feed additive and/or drinking water additive.

5. A feed additive and/or drinking water additive according to claim 4, **characterized in that** said inulin is contained in the feed additive in an amount of from 100 to 700 g/kg, particularly 320 to 540 g/kg.

6. A feed additive and/or drinking water additive according to claim 4, **characterized in that** said inulin is contained in the drinking water additive in an amount of from 530 to 950 g/kg, particularly 820 to 920 g/kg.

7. A feed additive and/or drinking water additive according to any one of the claims 1 to 6, **characterized in that** the bacteria of the strain *Streptococcus faecium* IMB 52 (DSM 3530) are partially replaced with at least one further bacterial strain.

8. A feed additive and/or drinking water additive according to any one of claims 1 to 7, **characterized in that** 10 to 50% of the bacteria of the strain *Streptococcus faecium* IMB 52 (DSM 3530) are replaced with at least one further bacterial strain.

9. A feed additive and/or drinking water additive according to any one of claims 1 to 8, **characterized in that** the at least one further bacterial strain is selected from the strains *Bifidobacterium thermophilum* (I-01) (DSM 14411), *Bifidobacterium thermophilum* (I-07) (DSM 14412), *Bifidobacterium boum* (I-12) (DSM 14413), *Bifidobacterium thermophilum* (I-15) (DSM 14414), *Bifidobacterium thermophilum* (I-19) (DSM 14415) und *Bifidobacterium thermophilum* (I-20) (DSM 14416).

10. A feed additive and/or drinking water additive according to any one of claims 1 to 9, **characterized in that** brown marine algae are additionally contained.

11. A feed additive and/or drinking water additive according to claim 9, **characterized in that** said brown algae are comprised of *Ascophyllum nodosum.*

12. A feed additive and/or drinking water additive according to claim 10 or 11, **characterized in that** *Ascophyllum nodosum* is contained in an amount of from 300 to 800 g/kg, particularly 450 to 550 g/kg, feed additive.

13. A feed additive and/or drinking water additive according to any one of claims 1 to 12, **characterized in that** 8 g *Bacillus subtilis,* 60,0 g *Streptococcus faecium* IMB 52 (DSM 3530), 432 g *Bifidobacterium thermophilum,* balance *Ascophyllum nodosum,* are contained per kg feed additive and/or drinking water additive.

14. A feed additive and/or drinking water additive according to any one of claims 1 to 12, **characterized in that** 10 g *Bacillus subtilis,* 24,75 *Streptococcus faecium* IMB 52 (DSM 3530), 10,5 g *Bifidobacterium thermophilum* (DSM 14414), balance inulin, particularly from chicory, are contained per kg feed additive and/or drinking water additive.

## Revendications

1. Additif alimentaire et/ou d'eau potable destiné à des animaux de rente, contenant au moins des bactéries de la souche *Streptococcus faecium* IMB 52 (DSM 3530), **caractérisé en ce qu'**il comprend en outre des composants de paroi cellulaire normalisés de *Bacillus* sp. et/ou de *Streptococcus* sp. et/ou de *Bifidobacterium* sp. et d'inuline.

2. Additif alimentaire et/ou d'eau potable selon la revendication 1, **caractérisé en ce que** les composants de paroi cellulaire normalisés sont choisis dans la souche *Bacillus subtilis.*

3. Additif alimentaire et/ou d'eau potable selon la revendication 1 ou 2, **caractérisé en ce que** les composants de paroi cellulaire normalisés de *Bacillus subtilis* sont choisis en une quantité de 0,2 à 40 g/kg d'additif alimentaire et/ou d'additif d'eau potable, en particulier, de 5 à 15 g/kg.

4. Additif alimentaire et/ou d'eau potable selon la revendication 1, **caractérisé en ce que** l'inuline est issue du poireau, de l'oignon, de l'ail, de l'artichaut, du blé, de la chicorée, du topinambour, de la tomate, de la banane et/ou de l'orge et est contenue en une quantité de 100 à 950 g/kg d'additif alimentaire et/ou d'eau potable.

5. Additif alimentaire et/ou d'eau potable selon la revendication 4, **caractérisé en ce que** l'inuline dans l'additif alimentaire est contenue en une quantité de 100 à 700 g/kg, en particulier, de 320 à 540 g/kg.

6. Additif alimentaire et/ou d'eau potable selon la revendication 4, **caractérisé en ce que** l'inuline dans l'additif d'eau potable est contenu en une quantité de 530 à 950 g/kg, en particulier de 820 à 920 g/kg.

7. Additif alimentaire et/ou d'eau potable selon l'une des revendications 1 à 6, **caractérisé en ce que** les bactéries de la souche *Streptococcus faecium* IMB 52 (DSM 3530) sont remplacées en partie par au moins une autre souche de bactéries.

8. Additif alimentaire et/ou d'eau potable selon l'une des revendications 1 à 7, **caractérisé en ce que** 10 à 50 % des bactéries de la souche *Streptococcus faecium* IMB 52 (DSM 3530) sont remplacés par au moins une autre souche de bactérie.

9. Additif alimentaire et/ou d'eau potable selon l'une des revendications 1 à 8, **caractérisé en ce qu'**au moins une autre souche de bactérie est choisie parmi les souches *Bifidobacterium thermophilum* (I-01) (DSM 14411), *Bifidobacterium thermophilum* (I-07) (DSM 14412), *Bifidobacterium boum* (I-12) (DSM 14413), *Bifidobacterium thermophilum* (I-15) (DSM 14414), *Bifidobacterium thermophilum* (I-19) (DSM 14415) et *Bifidobacterium thermophilum* (I-20) (DSM 14416).

10. Additif alimentaire et/ou d'eau potable selon l'une des revendications 1 à 9, **caractérisé en ce qu'**il contient en outre des algues brunes.

11. Additif alimentaire et/ou d'eau potable selon la revendication 9, **caractérisé en ce que** l'algue brune est *l'Ascophyllum nodosum.*

12. Additif alimentaire et/ou d'eau potable selon la revendication 10 ou 11, **caractérisé en ce que** *l'Ascophyllum nodosum* est contenue en une quantité de 300 à 800 g/kg, en particulier, 450 à 550 g/kg d'additif alimentaire.

13. Additif alimentaire et/ou d'eau potable selon l'une des revendications 1 à 12, **caractérisé en ce que** le produit contient par kg d'additif alimentaire et/ou d'eau potable, 8 g de *Bacillus subtilis,* 60,0 g de *Streptococcus faecium* IMB 52 (DSM 3530), 432 g de *Bifidobacterium thermophilum* et le reste est constitué par *l'Ascophyllum nodosum.*

14. Additif alimentaire et/ou d'eau potable selon l'une des revendications 1 à 12, **caractérisé en ce que** le produit contient par kg d'additif alimentaire et/ou d'eau potable, 10 g de *Bacillus subtilis,* 24,75 g de *Streptococcus faecium* IMB 52 (DSM 3530), 10,5 g de *Bifidobacterium thermophilum* et le reste est constitué par l'inuline, en particulier, de chicotée.
